# EUROPÄISCHE PATENTSCHRIFT

(11) **EP 3 773 241 B1**
(45) Veröffentlichungstag und Bekanntmachung des Hinweises auf die Patenterteilung: **15.12.2021**
(21) Anmeldenummer: 19725985.6
(22) Anmeldetag: 21.05.2019
(51) Int. Cl.: A61B 17/00, A61B 17/30, A61B 17/28, A61C 3/00, B25G 1/10, A61B 17/3201, A61C 3/10

(54) **MEDIZINISCHES INSTRUMENT**
MEDICAL INSTRUMENT
INSTRUMENT MÉDICAL

(30) Priorität: 23.05.2018 DE 102018112346
(43) Veröffentlichungstag der Anmeldung: 17.02.2021
(73) Patentinhaber: Storz am Mark GmbH, 78576 Emmingen-Liptingen (DE)
(72) Erfinder: MAZZEO, Martin, 78576 Emmingen-Liptingen (DE)
(74) Vertreter: Müller Schupfner & Partner Patent- und Rechtsanwaltspartnerschaft mbB
(86) Internationale Anmeldenummer: PCT/EP2019/063090
(87) Internationale Veröffentlichungsnummer: WO 2019/224196

(56) Entgegenhaltungen:
- WO-A1-2012/161782
- WO-A1-2015/100325
- WO-A2-2012/109594
- DE-A1-102007 047 059
- US-A1- 2006 257 820
- US-A1- 2013 247 333
- US-A1- 2014 005 661
- US-A1- 2017 224 399
- US-S1- D 419 851
- US-S1- D 419 852
- US-S1- D 433 914

## Beschreibung

Die vorliegende Erfindung betrifft ein medizinisches Instrument (insbesondere ein zahnmedizinisches Instrument) mit einem ersten Arm, der sich in Längsrichtung erstreckt und zwischen einem hinteren Ende und einem vorderen Arbeitsende einen Griffbereich aufweist.

Bei solchen Instrumenten ist es wichtig, dass ein Benutzer das medizinische Instrument gut und sicher d.h. ohne abzurutschen halten kann.

US 2013/0247333 A1 zeigt ein medizinisches Instrument, das zwei Arme aufweist, an denen jeweils ein Griffbereich angeordnet ist. In jedem Griffbereich ist eine Reihe von Langlöchern angeordnet, die sich in einer Längsrichtung des Instruments erstreckt. Neben den Langlöchern quer zur Längsrichtung befinden sich Vorsprünge.

WO 2012/161782 A1 zeigt ein medizinisches Instrument mit zwei Armen, die scherenartig zueinander beweglich sind. Im Griffbereich weist das medizinische Instrument jeweils ein längliches Griffloch auf.

WO 2015/190325 A1 zeigt einen orthopädischen Schraubenzieher, der im Griffbereich eine Vielzahl von Aussparungen aufweist.

DE 10 2007 047 059 A1 zeigt ein Verfahren zur Herstellung einer Griffstruktur. Bei der Griffstruktur sind eine Vielzahl von Langlöchern nebeneinander angeordnet.

US 2006/0257820 A1 zeigt ein zahnmedizinisches Instrument mit Langlöchern im Griffbereich.

Es ist daher Aufgabe der Erfindung, ein medizinisches Instrument (insbesondere ein zahnmedizinisches Instrument) mit einem ersten Arm, der sich in Längsrichtung erstreckt und zwischen einem hinteren Ende und einem vorderen Arbeitsende einen Griffbereich aufweist, bereitzustellen, das von einem Benutzer sicher gehalten werden kann.

Die Erfindung ist im Anspruch 1 definiert. Vorteilhafte Weiterbildungen sind in den abhängigen Ansprüchen angegeben.

Das erfindungsgemäße medizinisches Instrument (insbesondere ein zahnmedizinisches Instrument) weist einen ersten Arm, der sich in Längsrichtung erstreckt und zwischen einem hinteren Ende und einem vorderen Arbeitsende einen Griffbereich umfasst, auf, wobei im Griffbereich mindestens zwei sich in Längsrichtung erstreckende Langlöcher ausgebildet sind, die in Längsrichtung zueinander versetzt angeordnet sind. Vorteilhafterweise sind die Langlöcher derart ausgestaltet, um einem Benutzer einen Halt gegen Abrutschen zu geben.

Durch das Vorsehen der Langlöcher kann in vorteilhafter Weise das Gewicht des medizinischen Instrumentes verringert werden. Auch ist die Handhabung des medizinischen Instrumentes verbessert.

So sorgen Langlöcher z.B. in vorteilhafter Weise dafür, dass ein Benutzer mit seinen Fingern, mit denen er den Griffbereich hält, deutlich weniger abrutscht im Vergleich zu bekannten medizinischen Instrumenten, die die erfindungsgemäße Anordnung von Langlöchern nicht aufweisen. Ferner ist eine Wiederaufbereitung des erfindungsgemäßen medizinischen Instrumentes gut möglich.

Insbesondere können die Enden (nachfolgend auch Längsenden genannt) der Langlöcher, die die Langlöcher in Längsrichtung begrenzen, dem Benutzer einen Halt gegen Abrutschen geben, so dass aufgrund der mindestens zwei Langlöcher verschiedene günstige Griffpositionen bereitgestellt sind, bei denen ein Abrutschen vermieden ist.

Aufgrund der Langlöcher liegt somit eine verbesserte Haptik vor, was beispielsweise bei Drehbewegungen, Zugbewegungen, Schiebebewegungen und/oder Druckbewegungen des medizinischen Instrumentes hilfreich ist. Unter der in Längsrichtung zueinander versetzten Anordnung der mindestens zwei Langlöcher wird hier insbesondere verstanden, dass mindestens die vorderen oder hinteren Längsenden von zwei Langlöcher in Längsrichtung zueinander versetzt angeordnet sind und somit nicht auf gleicher Höhe liegen.

Bei dem erfindungsgemäßen medizinischen Instrument können sich die mindestens zwei Langlöcher in Längsrichtung erstrecken. Bevorzugt erstrecken sie sich parallel zur Längsrichtung. Eines oder mehrere oder alle Langlöcher können jedoch auch einen Winkel mit der Längsrichtung einschließen, der größer als 0° und kleiner oder gleich als 90° ist.

Ferner ist es möglich, dass sich mindestens zwei Langlöcher kreuzen bzw. schneiden.

Mindestens zwei der Langlöcher können in Längsrichtung voneinander beabstandet sein. Mit anderen Worten können die Langlöcher in der Längsrichtung hintereinander angeordnet sein.

Ferner können mindestens zwei der Langlöcher auf einer Geraden liegen, wobei sich die Gerade vorzugsweise parallel zu der Längsrichtung erstreckt. Die mindestens zwei Langlöcher sind quer zur Längsrichtung zueinander versetzt angeordnet.

Mindestens eines der Langlöcher ist als Durchgangsloch ausgebildet. Ferner kann mindestens eines der Langlöcher als Nut ausgebildet sein.

Ferner kann mindestens eines der Längsenden der Langlöcher abgerundet sein.

Die mindestens zwei Langlöcher können gleiche Abmessungen aufweisen oder sich in ihrer Länge und/oder Breite unterscheiden. Alternativ können die Längsenden auch eckig oder dreieckig ausgebildet sein.

Der Griffbereich kann zwei oder drei oder mehrere Gruppen von jeweils mindestens zwei in Längsrichtung (insbesondere zwei oder drei) zueinander versetzt angeordnete Langlöcher aufweisen. Die Gruppen von Langlöcher können quer zur Längsrichtung zueinander versetzt ausgebildet sein.

Die Gruppen von jeweils mindestens zwei Langlöchern können insbesondere zueinander parallel verlaufen. Es ist jedoch auch möglich, dass die Gruppen von jeweils mindestens zwei Langlöchern einen Winkel miteinander einschließen und somit nicht zueinander parallel verlaufen. Der Winkel kann bevorzugt im Bereich von größer als 0° und kleiner oder gleich als 20° und insbesondere kleiner oder gleich als 15° oder 10° liegen.

Ferner kann mindestens eines der Langlöcher zwei zueinander parallel verlaufende Seiten aufweisen.

Ferner können im Griffbereich mehrere in Längsrichtung voneinander beabstandete Nuten ausgebildet sein, die quer zur Längsrichtung verlaufen, so dass zwischen zwei benachbarten Nuten ein erhabener bzw. vorstehender Bereich vorliegt. Damit wird die Haptik des Griffbereiches weiter verbessert.
Alternativ kann der Griffbereich so ausgebildet sein, dass er bis auf die Langlöcher eine glatte Oberfläche aufweist. Der Griffbereich kann entweder flach oder gebogen bzw. gekrümmt sein.

Der Griffbereich kann im Bereich der Langlöcher (bevorzugt in einem Schnitt quer zur Längsrichtung gesehen) bogenförmig ausgebildet sein. Damit kann das Gewicht des medizinischen Instruments verringert werden, da aufgrund der Bogenform eine benötige Festigkeit bzw. Steifigkeit des medizinischen Instruments erreicht ist und somit die Materialstärke reduziert sein kann.

Das medizinische Instrument kann genau den ersten Arm und somit einen einzigen Arm aufweisen. Man kann es in diesem Fall z. B. als einarmiges Instrument bezeichnen, wobei es das vordere Arbeitsende aufweist und daher einendig ist. Es ist jedoch auch möglich, dass das hintere Ende auch als Arbeitsende ausgebildet ist, so dass das medizinische Instrument dann als zweiendig bezeichnet werden kann.

Das medizinische Instrument kann auch als zweiarmiges Instrument ausgebildet sein, wobei es dann einen zweiten Arm, der sich in Längsrichtung erstreckt und zwischen einem hinteren Ende und einem vorderen Arbeitsende einen Griffbereich aufweist, umfasst. Die beiden Arme sind an ihren hinteren Enden und/oder an ihren vorderen Arbeitsenden miteinander verbunden. Bevorzugt können die beiden Arme durch einen Benutzer aufeinander zubewegt (d.h. zusammengedrückt) werden, wenn der Benutzer z. B. mit Daumen und Zeigefinger auf die beiden Griffbereiche drückt. Ein solches zweiarmiges medizinisches Instrument kann z. B. eine Zange, eine Zahnzange, ein Nadelhalter, eine Schere oder eine Pinzette sein.

Bei einem solchen zweiarmigen medizinischen Instrument können im Griffbereich des zweiten Arms mindestens zwei sich in Längsrichtung erstreckende Langlöcher ausgebildet sein, die in Längsrichtung zueinander versetzt angeordnet sind. Damit wird ein eventuelles Abrutschen des Benutzers wesentlich verringert.
Der Griffbereich des zweiten Arms kann in gleicher Weise wie der Griffbereich des ersten Arms weitergebildet werden. Die beiden Griffbereiche können gleich oder unterschiedlich ausgebildet sein.
Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Nachfolgend wird die Erfindung anhand von Ausführungsbeispielen unter Bezugnahme auf die beigefügten Zeichnungen, die ebenfalls erfindungswesentliche Merkmale offenbaren, noch näher erläutert. Diese Ausführungsbeispiele dienen lediglich der Veranschaulichung und sind nicht als einschränkend auszulegen. Beispielsweise ist eine Beschreibung eines Ausführungsbeispiels mit einer Vielzahl von Elementen oder Komponenten nicht dahingehend auszulegen, dass alle diese Elemente oder Komponenten zur Implementierung notwendig sind. Vielmehr können andere Ausführungsbeispiele auch alternative Elemente und Komponenten, weniger Elemente oder Komponenten oder zusätzliche Elemente oder Komponenten enthalten. Elemente oder Komponenten verschiedener Ausführungsbespiele können miteinander kombiniert werden, sofern nichts anderes angegeben ist. Modifikationen und Abwandlungen, welche für eines der Ausführungsbeispiele beschrieben werden, können auch auf andere Ausführungsbeispiele anwendbar sein. Zur Vermeidung von Wiederholungen werden gleiche oder einander entsprechende Elemente in verschiedenen Figuren mit gleichen Bezugszeichen bezeichnet und nicht mehrmals erläutert. Von den Figuren zeigen:
- Fig. 1: eine perspektivische Ansicht eines ersten Ausführungsbeispiels des erfindungsgemäßen medizinischen Instruments 1;
- Fig. 2: eine vergrößerte Draufsicht auf den Griffbereich 6 des ersten Arms 2 des Instruments 1 von Fig. 1;
- Fig. 3: eine vergrößerte Seitenansicht des Griffbereichs 6 des ersten Arms 2 des Instruments 1 von Fig. 1;
- Fig. 4: eine vergrößerte Rückansicht des Griffbereichs 6 des ersten Arms 2 des Instruments 1 von Fig. 1;
- Fig. 5: eine Schnittansicht des Griffbereichs 6 des ersten Arms 2 des Instruments 1 gemäß der Schnittlinie A-A in Fig. 4
- Fig. 6: eine Abwandlung des Griffbereichs 6 des ersten Arms 2 in gleicher Darstellung wie in Fig. 5;
- Fig. 7: eine Abwandlung des Griffbereichs 6 des ersten Arms 2 in gleicher Darstellung wie in Fig. 5;
- Fig. 8: eine schematische vergrößerte Ansicht des Langlochs 22;
- Fig. 9: eine schematische vergrößerte Ansicht des Langlochs 22 gemäß einer Abwandlung;
- Fig. 10-21: schematische Darstellungen von Abwandlungen des Griffbereichs 6 des ersten Arms 2 des Instruments 1 von Fig. 1;
- Fig. 22: eine perspektivische Ansicht eines zweiten Ausführungsbeispiels des erfindungsgemäßen medizinischen Instruments 1;
- Fig. 23: eine perspektivische Ansicht eines dritten Ausführungsbeispiels des erfindungsgemäßen medizinischen Instruments 1;
- Fig. 24: eine perspektivische Ansicht eines vierten Ausführungsbeispiels des erfindungsgemäßen medizinischen Instruments 1, und
- Fig. 25: eine perspektivische Ansicht eines fünften Ausführungsbeispiels des erfindungsgemäßen medizinischen Instruments 1.

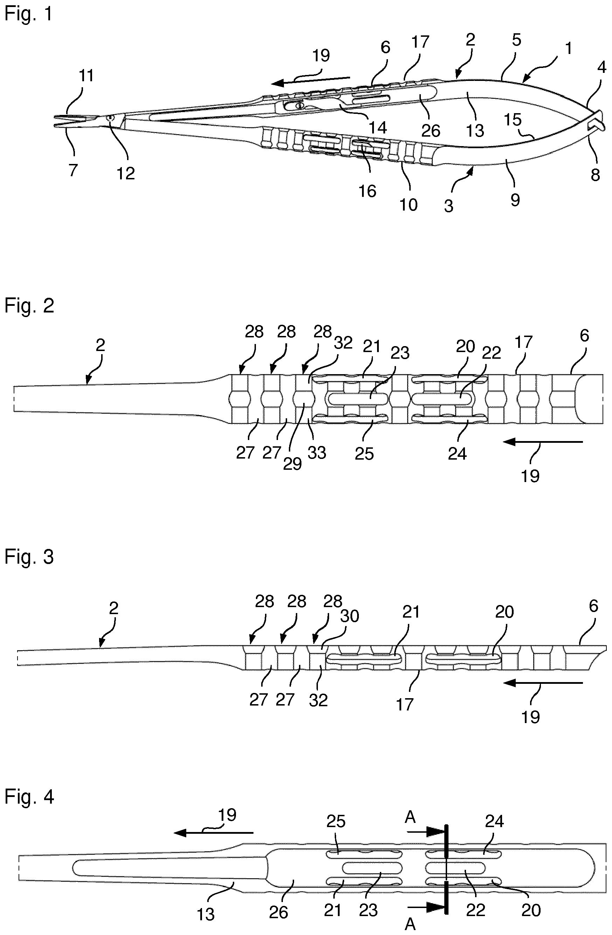

Bei dem in Fig. 1 gezeigten Ausführungsbeispiel ist das erfindungsgemäße medizinische Instrument 1, das insbesondere ein zahnmedizinisches Instrument 1 sein kann, als Nadelhalter ausgebildet und umfasst einen ersten Arm 2 sowie einen zweiten Arm 3.

Der erste Arm 2 umfasst ein erstes hinteres Ende 4, an dem sich ein erster Übergangsbereich 5 anschließt, an den ein erster Griffbereich 6 folgt. Vom ersten Griffbereich 6 erstreckt sich der erste Arm 2, der auch als erster Schenkel 2 bezeichnet werden kann, bis zu seinem ersten vorderen Arbeitsende 7, das hier als erste Greifbacke 7 ausgebildet ist.

In gleicher Weise umfasst der zweite Arm 3 ein zweites hinteres Ende 8, von dem sich ein zweiter Übergangsbereich 9 bis zu einem zweiten Griffbereich 10 erstreckt. Der zweite Griffbereich 10 geht dann in das zweite vordere Arbeitsende 11 über, das hier als zweite Greifbacke 11 ausgebildet ist.

Das erste hintere Ende 4 und das zweite hintere Ende 5 sind so ausgestaltet, dass eines der beiden Enden durch ein Durchgangsloch, das in dem anderen der beiden Enden ausgebildet ist, hindurchragt. Um zu verhindern, dass das eine Ende aus dem anderen Ende herausrutscht, ist eine Verdickung an dem äußersten Ende des Endes vorgesehen, das durch das Durchgangsloch hindurchragt. Mit anderen Worten sind das erste hintere Ende 4 und das zweite hintere Ende 5 ineinander gesteckt und somit beweglich miteinander verbunden.

Um die Funktionalität des Nadelhalters 1 bereitzustellen, kreuzen sich die beiden Arme 2 und 3 vor den vorderen Arbeitsenden 7, 11, wobei die Arme im Kreuzungsbereich über ein Drehgelenk 12 miteinander verbunden sind. Bei der in Fig. 1 gezeigten Grundstellung sind die beiden Arbeitsenden 7, 11 in der geöffneten Stellung, so dass eine maulartige Greifstruktur vorliegt. Wenn die beiden Arme 2, 3, deren hintere Enden 4, 8 wie oben beschrieben miteinander verbunden sind, durch einen Benutzer zusammengedrückt werden, was möglich ist, da die Übergangsbereiche 5, 9 elastisch ausgebildet sind, wird der Abstand der beiden Arme 2, 3 verringert und dadurch die maulartige Öffnung der Greifbacken 7, 11 geschlossen. Ein zwischen den Greifbacken 7, 11 vorher positionierter Gegenstand, wie z. B. eine Nadel, wird dabei durch die Greifbacken 7, 11 eingeklemmt. In dieser Stellung kann also ein Gegenstand zwischen den Greifbacken 7, 11 gehalten werden, weswegen diese Stellung als eine Haltestellung bezeichnet wird. Das Instrument 1 kann somit insbesondere als Nadelhalter bezeichnet werden. Es kann allgemein als medizinische Zange 1, medizinische Klemme 1 oder medizinisches Halteelement 1 bezeichnet werden. Hört der Benutzer auf die beiden Arme 2, 3 zusammenzudrücken, nimmt das medizinische Instrument 1 wieder die in Fig. 1 gezeigt Grundstellung ein. Mit anderen Worten bewirken insbesondere die elastisch ausgestalteten Übergangsbereiche 5, 9, dass eine Rückstellkraft wirkt, die das medizinische Instrument 1 in die Grundstellung zurückführt, in der die Greifbacken 7, 11 geöffnet sind.

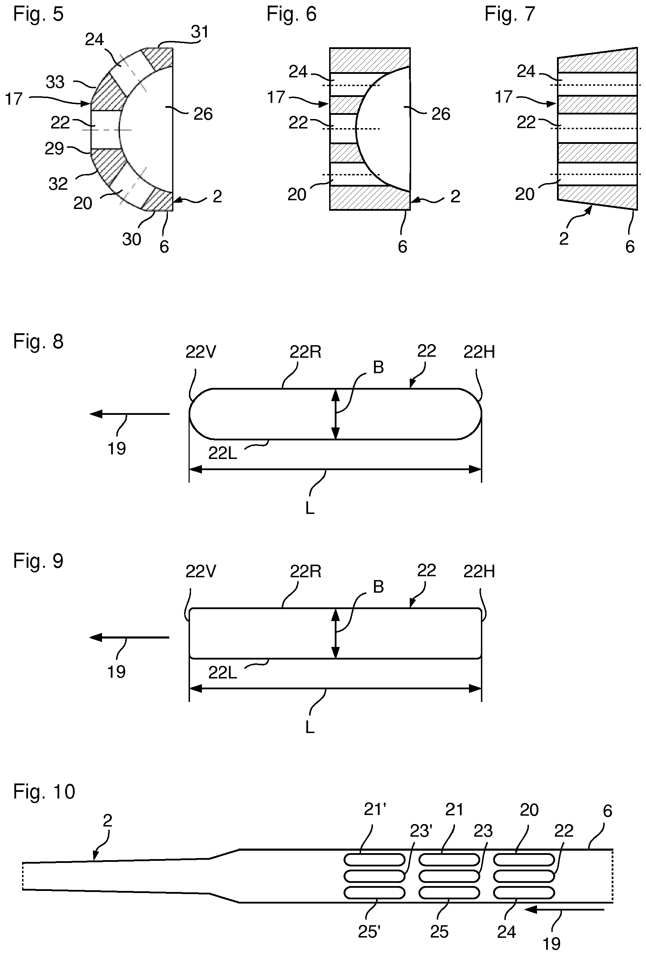

In Fig. 2 bis 4 sind vergrößerte Darstellungen des ersten Griffbereiches 6 des ersten Arms 2 gezeigt, um die erfindungsgemäße Ausbildung des ersten Griffbereiches 6 näher erläutern zu können. In Fig. 5 ist der Schnitt A-A gemäß der Schnittlinie aus Fig. 4 dargestellt.

Wie in Fig. 1 ersichtlich ist, ist an der Innenseite 13 des ersten Arms 2 ein erstes Sperrelement 14 befestigt, das von der Innenseite 13 in Richtung des zweiten Arms 3 vorsteht. Auf der Innenseite 15 des zweiten Arms 3 ist ein entsprechendes bzw. zu dem ersten Sperrelement 14 komplementäres zweites Sperrelement 16 befestigt, das durch ein Langloch des zweiten Griffbereiches bei der Darstellung in Fig. 1 zum Teil sichtbar ist. Befindet sich das medizinische Instrument 1 in der Haltestellung und drückt der Benutzer die beiden Arme 2, 3 weiter zusammen rastet das zweite Sperrelement 16 mit dem ersten Sperrelement 14 so ein, dass die Haltestellung fixiert bzw. gehalten ist, auch wenn der Benutzer die beiden Arme 2, 3 nicht mehr zusammendrück, so dass ein sicheres Greifen mit den beiden Greifbacken 7 und 11 möglich ist. Dabei wird von den beiden Greifbacken 7, 11 eine konstante, voreinstellbare Haltekraft auf den zu haltenden Gegenstand ausgeübt. Insbesondere ist die Haltekraft durch das erste und zweite Sperrelement 14, 16 einstellbar indem durch das erste und zweite Sperrelement 14, 16 definiert ist, wie nah die beiden Arme 2, 3 beieinander in der Haltestellung gehalten werden. Mit anderen Worten kann ein Benutzer durch Zusammendrücken der beiden Arme 2, 3 das erste Sperrelement 14 mit dem zweiten Sperrelement 16 so in Eingriff bringen, dass die Haltestellung beibehalten wird, auch wenn der Benutzer das medizinische Instrument 1 beispielsweise aus der Hand legt. Befindet sich das medizinische Instrument 1 in der Haltestellung, so dass die beiden Greifbacken 7, 11 geschlossen sind, kann der Benutzer durch ein weiteres Zusammendrücken der beiden Arme 2, 3 das erste Sperrelement 14 von dem zweiten Sperrelement 16 lösen, so dass das medizinische Instrument 1 von den Sperrelementen nicht mehr in der Haltestellung gehalten wird. Hört der Benutzer dann auf die beiden Arme 2, 3 zusammenzudrücken kann das medizinische Instrument 1 also wieder in die Grundstellung zurückkehren.
Die Sperrelemente 14, 16 sind jedoch nicht zwingend notwendig und können auch weggelassen werden. Daher ist das erste Sperrelement 14 in den Darstellungen gemäß Fig. 2 bis 5 nicht eingezeichnet.

Da der zweite Griffbereich 10 grundsätzlich genauso ausgebildet ist wie der erste Griffbereich 6, wird nachfolgend nur der erste Griffbereich 6 im Detail beschrieben.

Um ein Abrutschen der Finger des Benutzers bei der Benutzung des medizinischen Instrumentes 1 zu verhindern, weist der erste Griffbereich 6 drei Paare von jeweils zwei in Längsrichtung 19 des ersten Arms 2 hintereinander angeordneten Langlöchern 20, 21; 22, 23; 24, 25 auf. Jedes Paar von Langlöchern 20, 21; 22, 23; 24 und 25 ist bei dem hier beschriebenen Ausführungsbeispiel so angeordnet, dass die Langlöcher 20-25 jedes Paares mit ihren Längsachsen auf einer Geraden liegen, die parallel zur Längsrichtung 19 verläuft. Die Langlöcher 20-25 sind insbesondere dadurch gekennzeichnet, dass ihre Ausdehnung L in Längsrichtung (wie in Fig. 6 für das vergrößert dargestellte Langloch 22 ersichtlich ist) mindestens zweimal so groß ist wie ihre Ausdehnung B quer zur Längsrichtung 19. Bevorzugt ist die Ausdehnung des Langlochs in Längsrichtung 19 x-mal so groß wie die Ausdehnung des Langlochs quer zur Längsrichtung 19, wobei x im Bereich von 2 bis 70 und bevorzugt 2 bis 10 oder 5 bis 8 liegt. Die Breite B kann im Bereich von 1 mm bis 15 mm liegen. Insbesondere kann sie im Bereich von 1 mm bis 10 mm oder 1,5 mm bis 5 mm liegen. Es ist auch möglich, dass die Breite 2 mm beträgt. Die Länge L kann im Bereich von 2 mm bis 70 mm liegen. Bevorzugt ist ein Bereich von 2 mm bis 35 mm und insbesondere ein Bereich von 2 mm bis 26 mm. Die Länge L kann beispielsweise 20, 26, 35 oder 70 mm betragen.

Bevorzugt zeichnen sich die Langlöcher 20-25, wie in Fig. 8 für das Langloch 22 gezeigt ist, dadurch aus, dass sie zwei parallel zueinander verlaufende Längsseiten 22R und 22L aufweisen. Die Längsseiten 22R und 22L sind durch ein hinteres Ende 22H und ein vorderes Ende 22V miteinander verbunden. Die Ausdehnung der Längsseiten 22R, 22L beträgt bevorzugt mindestens 50% der Ausdehnung L in Längsrichtung 19. Bevorzugt kann die Ausdehnung der Längsseiten 22R, 22L mindestens 60%, 70%, 80%, 90% oder 95% der Ausdehnung L betragen. Es ist auch möglich, dass die Ausdehnung 22R, 22L praktisch 100% der Ausdehnung L beträgt, wie in Fig. 9 schematisch dargestellt ist. In diesem Fall sind das vordere und hintere Ende 22V und 22H nicht abgerundet, wie in Fig. 8 gezeigt ist, sondern verlaufen insbesondere senkrecht zur Erstreckungsrichtung der Längsseiten 22R, 22L.

Man kann auch sagen, dass die Ausdehnung B quer zur Längsrichtung 19 und somit die Breite B der Langlöcher 20-25 (bis eventuell auf das vordere und hintere Ende 22V, 22H) konstant ist.

Wie insbesondere der Schnittdarstellung in Fig. 5 entnommen werden kann, ist der erste Griffbereich 6 bogenförmig ausgebildet und weist einen näherungsweise kreisringabschnittsförmigen Querschnitt auf, wobei die Langlöcher 20-25 als durchgehende Löcher 20-25 ausgebildet sind, so dass sie sich vollständig durch die Wandung im ersten Griffbereich 6 erstrecken. Alternativ ist es möglich, die Langlöcher 20-25 nutförmig auszubilden, wobei sie dann lediglich zur ersten Außenseite 17 des ersten Arms 2 hin offen sind. Die Ausbildung als durchgehende Löcher ist jedoch insbesondere unter dem Gesichtspunkt der Reinigung, Desinfektion und/oder Sterilisation (insbesondere der Wiederaufbereitung) des medizinischen Instrumentes 1 von Vorteil.

Da die Querschnittsform des ersten Griffbereiches 6 grundsätzlich kreissegmentförmig ist, ist auf der ersten Innenseite 13 eine innere Ausnehmung 26 mit im Wesentlichen wiederum kreissegmentförmiger Querschnittsform ausgebildet, wie Fig. 5 entnommen werden kann. Mit anderen Worten ist der Griffbereich 6 bogenförmig ausgebildet, so dass er nach außen, das heißt von dem zweiten Arm 3 weg zeigend, gebogen ist bzw. sich ausbeult. Dadurch wird vorteilhaft ein geringes Gewicht des medizinischen Instrumentes 1 erreicht, da die benötigte Festigkeit bzw. Steifigkeit durch geometrische Ausformung des Griffbereichs 6 sichergestellt ist.

Aufgrund der inneren Ausnehmung 26 ist die erste Innenseite 13 des ersten Arms 2 im Bereich des ersten Griffbereiches 6 kontinuierlich gekrümmt. Die kontinuierliche Krümmung ist nur durch die Langlöcher 20 bis 25 unterbrochen.
In den in Figuren 6 und 7 gezeigten Abwandlungen kann der erste Griffbereich 6 als sogenannter Flachgriff ausgebildet sein, so dass keine kontinuierliche Krümmung vorliegt. Die Querschnittsform kann beispielsweise rechteckig (Figur 6) oder trapezförmig (Figur 7) sein.

Um die Haptik des ersten Griffbereiches 6 weiter zu verbessern, sind auf der Außenseite 17 bogenförmige Nuten 27 ausgebildet, die sich quer zur Längsrichtung 19 erstrecken. Die Nuten 27 sind in Längsrichtung 19 voneinander beabstandet, so dass zwischen den Nuten 27 erhabene bogenförmige Bereiche 28, die relativ zu den Nuten 27 erhaben sind bzw. vorstehen, ausgebildet sind. Jeder erhabene Bereich 28 weist einen mittleren planen Abschnitt 29 und davon quer zur Längsrichtung 19 beabstandet zwei seitliche plane Abschnitte 30 und 31 auf. Die planen Abschnitte 29, 30 und 31 sind durch gekrümmt verlaufende Zwischenabschnitte 32 und 33 verbunden.

Wie der Darstellung in Fig. 2 entnommen werden kann, erstrecken sich das dritte und vierte Langloch 22, 23 durch die mittleren planen Abschnitte 29 der entsprechenden erhabenen Bereiche 28, so dass die Langlöcher 22, 23 und die mittleren planen Abschnitte 29 auf einer Ebene liegen und der Benutzer problemlos mit den Langlöchern 22, 23 in Kontakt kommen kann. Das erste und zweite Langloch 20, 21 sowie das fünfte und sechste Langloch 24, 25 erstrecken sich durch die gekrümmten Zwischenabschnitte 32 bzw. 33.

Durch die beschriebene Ausbildung des ersten Griffbereiches 6 und die entsprechende Ausbildung des zweiten Griffbereiches 10 kann der Benutzer somit das medizinische Instrument 1 sicher z. B. zwischen seinem Daumen und Zeigefinger halten und benutzen. Ein Abrutschen in Längsrichtung 19 wird insbesondere durch die Langlöcher 20 bis 25 verhindert. Da mindestens zwei Langlöcher 20, 21; 22, 23; 24, 25 in Längsrichtung 19 hintereinander angeordnet sind, gibt es für die Benutzer verschiedene optimale Greifpositionen auf den Griffbereichen 6, 10. Bevorzugt kann der Benutzer die Griffbereiche 6, 10 an den zu den vorderen Enden 7, 11 weisenden Enden der Langlöcher 20-25 halten.

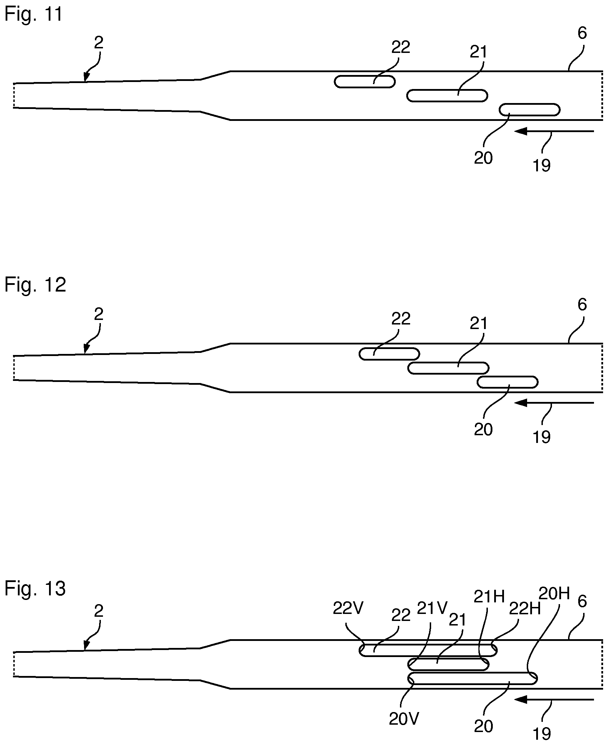

Die Ausbildung von drei Paaren mit jeweils zwei Langlöchern 20-25 pro Griffbereich 6, 10 ist rein beispielhaft zu verstehen. Wesentlich ist, dass pro Griffbereich 6, 10 mindestens zwei in Längsrichtung 19 zueinander versetzt (hier hintereinander) angeordnete Langlöcher 20, 21; 22, 23; 24,25 vorgesehen sind. Es können also auch z. B. drei oder vier Langlöcher hintereinander vorgesehen sein. Die hintereinander angeordneten Langlöcher können so, wie bisher beschrieben, auf einer Linie hintereinander angeordnet sein. So können, wie in der schematischen Darstellung von Fig. 10 ersichtlich ist, drei Gruppen von jeweils drei hintereinander angeordneten Langlöchern 20, 21, 21'; 22, 23, 23' sowie 24, 25 und 25' angeordnet sein. Die Paare bzw. Gruppen von hintereinander angeordneten Langlöchern können insbesondere parallel zueinander angeordnet sein. Es ist jedoch auch möglich, dass ein seitlicher Versatz vorliegt, wie schematisch in Fig. 11 dargestellt ist.

Ferner ist es auch nicht zwingend erforderlich, dass die mindestens zwei Langlöcher 20, 21 in Längsrichtung voneinander beabstandet sind, wie dies bei den bisher beschriebenen Ausführungsbeispielen der Fall war. Es ist auch möglich, dass in Längsrichtung 19 eine teilweise Überlappung (aber keine vollständige Überlappung) vorliegt, wie dies für die Langlöcher 20, 21 und 22 in Fig. 12 gezeigt ist. Des Weiteren können die Langlöcher so in Längsrichtung 19 zueinander versetzt angeordnet sein, dass die vorderen und/oder hinteren Enden von zwei Langlöchern in Längsrichtung 19 zueinander versetzt bzw. voneinander beabstandet sind. Dies gilt z. B. für die hinteren Enden 20H und 21H der beiden Langlöcher 20 und 21 bei dem Ausführungsbeispiel von Fig. 13. Die vorderen Enden 20V und 21V der beiden Langlöcher liegen in Längsrichtung 19 auf gleicher Höhe.

Bei den beiden Langlöchern 21 und 22 gemäß Fig. 13 sind sowohl die vorderen Enden 21V und 22V als auch die hinteren Enden 21H und 22H in Längsrichtung 19 zueinander versetzt, obwohl das Langloch 22 das Langloch 21 in Längsrichtung 19 vollständig überlappt. Dies ist hier der Fall, da die beiden Langlöcher 21 und 22 unterschiedlich lang sind.

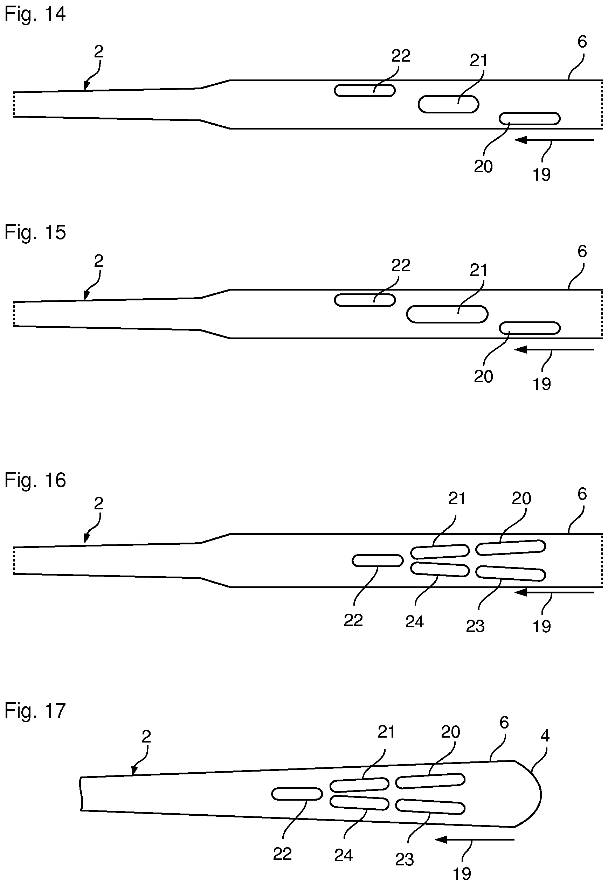

Natürlich können auch bei den anderen beschriebenen Ausführungsbeispielen in einer Abwandlung die Langlöcher 20-25 unterschiedlich lang in Längsrichtung 19 und/oder unterschiedlich breit (Ausdehnung quer zur Längsrichtung 19) ausgebildet sein, wie z. B. in Fig. 14 und 15 gezeigt ist. Beim Ausführungsbeispiel von Fig. 14 ist das Langloch 21 breiter als die anderen beiden Langlöcher 20 und 22 und beim Ausführungsbeispiel von Fig. 15 ist das Langloch 21 sowohl breiter als auch länger als die anderen beiden Langlöcher 20 und 22.

Natürlich ist es bei den Ausführungsbeispielen gemäß Fig. 14 und 15 auch möglich, dass das Langloch 20 und/oder 22 breiter sind/ist als das Langloch 21. Bei den bisher beschriebenen Ausführungsbeispielen erstrecken sich die Langlöcher 20 - 25 stets parallel zur Längsrichtung 19. Dies ist nicht zwingend notwendig. Wie bei dem Ausführungsbeispiel von Fig. 16 schematisch dargestellt ist, sind die Langlöcher 20 und 21 gegenüber der Längsrichtung 19 um einen gewissen Winkel geneigt angeordnet. Dieser Winkel kann beispielsweise im Bereich von 2° - 7° liegen. Die Langlöcher 23 und 24 sind dann um den entsprechenden negativen Winkel angeordnet. Die Anordnung der Langlöcher 20, 21 sowie 23, 24 kann auch als V-förmig bezeichnet werden. Ferner ist noch das Langloch 22 angeordnet, das sich parallel zur Längsrichtung 19 erstreckt.
In Fig. 17 ist die gleiche Anordnung der Langlöcher 20 - 24 ausgebildet, jedoch ist der Griffbereich 6 mit abnehmbarer Breite in Längsrichtung 19 gestaltet.

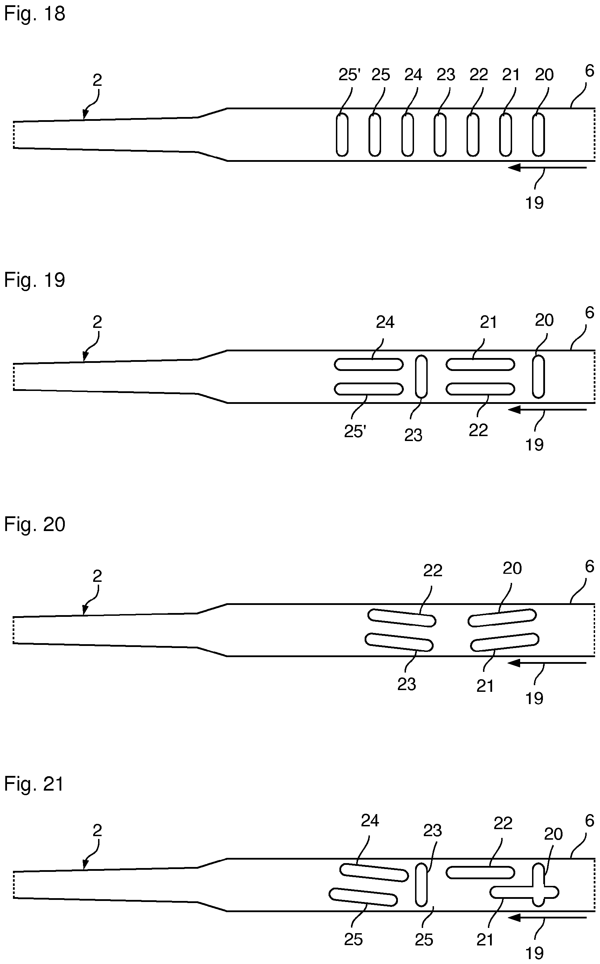

Es ist auch möglich, dass sich die Langlöcher quer zur Längsrichtung erstrecken, wie schematisch in dem Ausführungsbeispiel gemäß Figur 18 gezeigt ist. In diesem Fall erstrecken sich die Langlöcher 20 - 25, 25' senkrecht zur Längsrichtung 19. Es ist jedoch auch jeder andere Winkel aus dem Bereich von 0° - 90° möglich.

Ferner können die Langlöcher auch abwechselnd senkrecht zur Längsrichtung 19 und parallel zur Längsrichtung 19 angeordnet sein, wie in Figur 19 schematisch dargestellt ist.

In Figur 20 sind unterschiedliche Winkel zur Längsrichtung 19 für die Langlöcher 20, 21 sowie 22, 23 dargestellt.

In Figur 21 ist ein Ausführungsbeispiel gezeigt, bei dem sich die Langlöcher 20 und 21 kreuzen bzw. schneiden. Ferner sind noch weitere Langlöcher 22, 23, 24 und 25 eingezeichnet, die unterschiedliche Winkel zur Längsrichtung 19 aufweisen.

Die Anordnung und Ausbildung der Langlöcher gemäß Figuren 10-21 sind rein beispielhaft zu verstehen und es ist möglich, die in diesen Figuren gezeigten Varianten oder Teile davon miteinander zu kombinieren.

Bei den Ausführungsbeispielen gemäß Fig. 10 bis 21 sind die Nuten 27 und erhabenen Bereiche 28 im Vergleich zum Ausführungsbeispiel gemäß Fig. 1 bis 5 nicht vorgesehen. Es ist jedoch optional möglich, in gleicher Weise wie beim Ausführungsbeispiel gemäß Fig. 1 bis 5 bei den Varianten gemäß Fig. 10 bis 21 solche Nuten 27 und erhabene Bereiche 28 auszubilden.

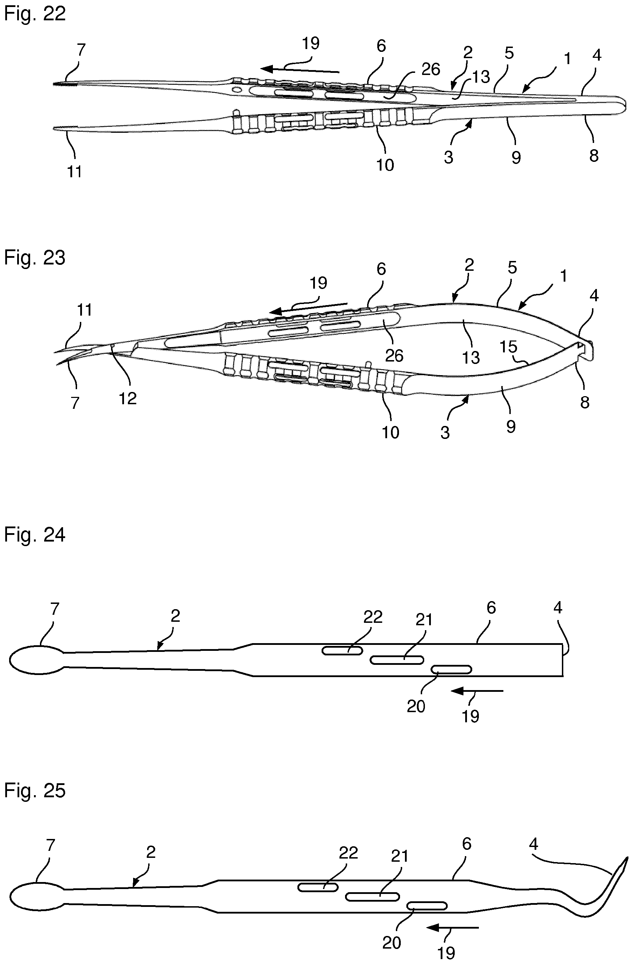

Das medizinische Instrument 1 kann z. B. auch als Pinzette ausgebildet sein, wie in Fig. 22 gezeigt ist. Bei der Pinzette gemäß Fig. 22 sind in gleicher Weise wie bei dem Nadelhalter gemäß Fig. 1 bis 5 Langlöcher 20-25 im ersten und zweiten Griffbereich 6, 10 des ersten und zweiten Arms 2, 3 ausgebildet. Lediglich die Verbindung der hinteren Enden 4, 8 und die Ausbildung der vorderen Enden 7, 11 ist unterschiedlich, um die gewünschte Funktionalität einer Pinzette bereitzustellen. So sind in diesem Fall die hinteren Enden 4, 8 fest miteinander verbunden.

In Fig. 23 ist eine Schere 1 gezeigt, die sich im Wesentlichen von dem Nadelhalter 1 gemäß Fig. 1 durch die Ausbildung der Arbeitsenden 7, 11 unterscheidet. Wesentlich hierbei ist wiederum, dass die Griffbereiche 6, 10 der beiden Arme 2, 3 in gleicher Weise ausgebildet sind.

Das medizinische Instrument 1 kann eine Ausdehnung in Längsrichtung 19 beispielsweise im Bereich von 4 bis 30 cm und insbesondere im Bereich von 10 bis 25 cm oder 15-20 cm aufweisen. So kann die Länge beispielsweise 16,5 cm, 17,5 cm 18 cm oder 20 cm betragen. Die maximale Abmessung eines Arms 2, 3 quer zur Längsrichtung 19 kann im Bereich von 5 bis 30 mm und insbesondere im Bereich von 7 bis 20 mm liegen. So kann die Breite des Arms 2, 3 beispielsweise 7,5 mm, 10 mm oder 18 mm betragen.

Als Material kann insbesondere Kunststoff, Keramik, Metall, Stahl, rostfreier Stahl und/oder Titan verwendet werden. Natürlich können auch bekannte Metalllegierungen verwendet werden.

Bei den bisher beschriebenen Ausführungsbeispielen sind die beiden Griffbereiche 6 und 10 gleich ausgebildet (insbesondere hinsichtlich der Langlöcher 20-25). Dies ist aber nicht zwingend erforderlich. Natürlich können die beiden Griffbereiche 6 und 10 unterschiedlich ausgebildet sein. Diese unterschiedliche Ausbildung kann insbesondere die Langlöcher 20-25 betreffen. So kann z. B. die Anzahl, die Anordnung und/oder die Abmessungen der Langlöcher 20-25 bei den beiden Griffbereichen 6, 10 unterschiedlich sein.

Bei den bisher beschriebenen Ausführungsbeispielen weist das medizinische Instrument 1 stets zwei Arme 2, 3 (bzw. zwei Schenkel 2, 3) auf. Natürlich ist es auch möglich, dass das medizinische Instrument 1 nur einen Arm 2 aufweist, wie bei dem Ausführungsbeispiel in Fig. 24 gezeigt ist. Das vordere Arbeitsende 7 ist löffelartig ausgebildet und im Griffbereich 6 sind drei in Längsrichtung 19 zueinander versetzt angeordnete Langlöcher 20, 21 und 22 ausgebildet. Die Langlöcher 20-21 sind hier als Durchgangslöcher ausgebildet. In einer Abwandlung können sie auch als Nuten 20-22 und somit als Längsnuten 20-22 ausgebildet sein.

Der Griffbereich 6 kann hohl ausgebildet sein. Es ist aber auch eine nicht hohle Ausbildung, so dass der Griff als voll bezeichnet werden kann, möglich.

Bei der Ausführungsform von Fig. 24 weist das medizinische Instrument ein Arbeitsende 7 auf und kann daher auch als einendiges Instrument 1 bezeichnet werden. Natürlich ist es auch möglich, das medizinische Instrument 1 mit nur einem Arm 2 und zwei Arbeitsenden 4, 7 auszubilden, wie in Fig. 25 gezeigt ist.

Das erste Ende 4 kann als erstes Arbeitsende 4 (hier als gebogene Spitze 4) und das vordere Ende 7 kann als zweites Arbeitsende 7, das wiederrum löffelartig ausgebildet ist, bezeichnet werden. Bei dieser Ausführungsform weist der Griffbereich 6 drei in Längsrichtung 19 zueinander versetzt angeordnete Langlöcher 20, 21 und 22 auf.

Natürlich können die Griffbereiche 6 der medizinischen Instrumente 1 gemäß Fig. 24 und 25 auch alle in Bezug mit den Figuren 1 bis 23 beschriebenen Anordnungen von Langlöchern 20-25 aufweisen. Ferner können die Griffbereiche der medizinischen Instrumente 1 gemäß Fig. 24 und 25 auch die beschriebene Struktur aus Nuten 27 und erhabenen Bereiche 28 aufweisen.

Bei den bisher beschriebenen Ausführungsformen sind zumindest die Griffbereiche 6, 10 stets sich geradlinig erstreckend ausgebildet. Es ist jedoch auch möglich, dass die Griffbereiche 6, 10 einen gekrümmten Verlauf in Richtung vom hinteren zum vorderen Ende 4, 7 aufweisen.

Das erfindungsgemäße medizinische Instrument kann mit einem sogenannten Fingergriff oder ohne Fingergriff und somit neutral ausgebildet sein. Dies trifft für die einarmige und zweiarmige Ausführung zu. Wenn der Arm als Fingergriff ausgebildet ist, ist eine Vorformung für die Finger der rechten Hand ausgebildet, so dass eine entsprechende Betätigung mit der rechten Hand vorgegeben ist. Die Ausbildung als Fingergriff wird häufig angeboten, damit ein Abrutschen sicher vermieden werden kann. In diesem Fall ist aber das medizinische Instrument, wie z.B. eine zahnmedizinische Zange, nur mit der rechten Hand zu bedienen. Durch das Vorsehen der Langlöcher kann jedoch auf die Ausbildung als Fingergriff verzichtet werden, da durch die Langlöcher ein Abrutschen sicher verhindert werden kann. Damit kann das medizinische Instrument (z.B. die zahnmedizinische Zange) sowohl von Rechtshändern als auch von Linkshändern sicher benutzt werden. Des Weiteren ist die Wiederaufbereitung des medizinischen Instrumentes gut möglich.

## Patentansprüche

1. Zahnmedizinisches Instrument mit
einem ersten Arm (2, 3), der sich in Längsrichtung (19) erstreckt und zwischen einem hinteren Ende (4, 8) und einem vorderen Arbeitsende (7, 11) einen Griffbereich (6, 10) aufweist,
wobei im Griffbereich mindestens zwei Langlöcher (20, 21, 22, 23, 24, 25) ausgebildet sind, die in Längsrichtung (19) zueinander versetzt angeordnet sind,
wobei die mindestens zwei Langlöcher (20-25) quer zur Längsrichtung (19) zueinander versetzt angeordnet sind, **dadurch gekennzeichnet, dass** mindestens eines der Langlöcher (20-25) als Durchgangsloch ausgebildet ist.

2. Zahnmedizinisches Instrument nach Anspruch 1, bei dem sich die mindestens zwei Langlöcher (20-25) in Längsrichtung erstrecken.

3. Zahnmedizinisches Instrument nach Anspruch 1 oder 2, bei dem die mindestens zwei Langlöcher (20-25) in Längsrichtung (19) voneinander beabstandet sind.

4. Zahnmedizinisches Instrument nach einem der obigen Ansprüche, bei dem bei mindestens einem der Langlöcher (20-25) die beiden Enden (20V, 21V, 22V, 20H, 21H, 22H) des Langlochs (20-25) abgerundet sind.

5. Zahnmedizinisches Instrument nach einem der obigen Ansprüche, bei dem sich zwei der Langlöcher (20-25) in ihrer Ausdehnung in Längsrichtung (19) und/oder in ihrer Ausdehnung quer zur Längsrichtung (19) unterscheiden.

6. Zahnmedizinisches Instrument nach einem der obigen Ansprüche, bei dem im Griffbereich (6, 10) mindestens zwei Gruppen von jeweils mindestens zwei Langlöchern (20-25) ausgebildet sind,
wobei die Langlöcher (20-25) jeder Gruppe in Längsrichtung (19) zueinander versetzt angeordnet sind,
wobei die Gruppen quer zur Längsrichtung (19) voneinander beabstandet sind, und
wobei die mindestens zwei Langlöcher (20-25) jeder Gruppe auf einer Geraden liegen.

7. Zahnmedizinisches Instrument nach Anspruch 6, bei dem die Gruppen von jeweils mindestens zwei Langlöchern zueinander parallel verlaufen oder einen Winkel miteinander einschließen, der im Bereich von größer als 0° und kleiner oder gleich als 20° liegt.

8. Zahnmedizinisches Instrument nach einem der obigen Ansprüche, bei dem im Griffbereich (6, 10) mehrere in Längsrichtung (19) voneinander beabstandete Nuten (27) ausgebildet sind, die quer zur Längsrichtung (19) verlaufen, so dass zwischen zwei benachbarten Nuten (27) ein erhabener Bereich (28) vorliegt.

9. Zahnmedizinisches Instrument nach einem der obigen Ansprüche, bei dem der Griffbereich (6, 10) im Bereich der Langlöcher (20-25) bogenförmig ausgebildet ist.

10. Zahnmedizinisches Instrument nach einem der obigen Ansprüche, bei dem mindestens eines der Langlöcher (20-25) zwei zueinander parallel verlaufende Seiten (22L, 22R) aufweist.

11. Zahnmedizinisches Instrument nach einem der obigen Ansprüche, bei dem ein zweiter Arm (2, 3), der sich in Längsrichtung (19) erstreckt und zwischen einem hinteren Ende (4, 8) und einem vorderen Arbeitsende (7, 11) einen Griffbereich (6, 10) aufweist, vorgesehen ist,
wobei beide Arme (2,3) *an ihren hinteren Enden (4, 8) und*/*oder ihren vorderen Arbeitsenden (7,* 11) miteinander verbunden sind, und
wobei im Griffbereich (10) des zweiten Arms (2, 3) mindestens zwei sich in Längsrichtung (19) erstreckende Langlöcher (20, 21, 22, 23, 24, 25) ausgebildet sind, die in Längsrichtung (19) zueinander versetzt angeordnet sind.

## Claims

1. Dental instrument having
a first arm (2, 3) which extends in the longitudinal direction (19) and has a grip region (6, 10) between a rear end (4, 8) and a front working end (7, 11)
wherein at least two elongated holes (20, 21, 22, 23, 24, 25) are formed in the grip region, which are arranged offset from one another in the longitudinal direction (19),
wherein the at least two elongated holes (20-25) are arranged offset to each other transversely to the longitudinal direction (19),
**characterized in that**
at least one of the elongated holes (20-25) is formed as a through hole.

2. Dental instrument according to claim 1, wherein the at least two elongated holes (20-25) extend in the longitudinal direction.

3. Dental instrument according to claim 1 or 2, wherein the at least two elongated holes (20-25) are spaced apart from each other in the longitudinal direction (19).

4. Dental instrument according to any one of the preceding claims, wherein in at least one of the elongated holes (20-25), the two ends (20V, 21V, 22V, 20H, 21H, 22H) of the elongated hole (20-25) are rounded.

5. Dental instrument according to any one of the preceding claims, wherein two of the elongated holes (20-25) differ in their extension in the longitudinal direction (19) and/or in their extension transversely to the longitudinal direction (19).

6. Dental instrument according to any one of the above claims, in which at least two groups of at least two elongated holes (20-25) are formed in the grip region (6, 10),
wherein the elongated holes (20-25) of each group are arranged offset to each other in the longitudinal direction (19),
the groups being spaced apart from each other transversely to the longitudinal direction (19), and
wherein the at least two elongated holes (20-25) of each group lie on a straight line.

7. Dental instrument according to claim 6, wherein the groups of at least two elongated holes each are parallel to each other or include an angle with each other which is in the range of greater than 0° and less than or equal to 20°.

8. Dental instrument according to any of the preceding claims, in which a plurality of longitudinally spaced grooves (27) are formed in the grip region (6, 10) and extend transversely to the longitudinal direction (19) so that a raised region (28) is present between two adjacent grooves (27).

9. Dental instrument according to any one of the preceding claims, wherein the grip region (6, 10) is arcuate in the region of the elongated holes (20-25).

10. Dental instrument according to any one of the preceding claims, wherein at least one of the elongated holes (20-25) has two sides (22L, 22R) extending parallel to each other.

11. Dental instrument according to any one of the preceding claims, wherein a second arm (2, 3) extending longitudinally (19) and having a grip portion (6, 10) between a rear end (4, 8) and a front working end (7, 11) is provided, both arms (2, 3) being connected to one another at their rear ends (4, 8) and/or their front working ends (7, 11), and
wherein at least two elongated holes (20, 21, 22, 23, 24, 25) are formed in the grip region (10) of the second arm (2, 3), which elongated holes extend in the longitudinal direction (19) and are arranged offset relative to one another in the longitudinal direction (19).

## Revendications

1. Instrument dentaire, comprenant
un premier bras (2, 3) qui s'étend en direction longitudinale (19) et qui présente une zone de poignée (6, 10) entre une extrémité arrière (4, 8) et une extrémité de travail avant (7, 11),
dans lequel
au moins deux trous oblongs (20, 21, 22, 23, 24, 25) sont ménagés dans la zone de poignée, qui sont disposés de manière décalée l'un par rapport à l'autre en direction longitudinale (19),
lesdits au moins deux trous oblongs (20-25) sont disposés de manière décalée l'un par rapport à l'autre transversalement à la direction longitudinale (19),
**caractérisé en ce que** l'un au moins des trous oblongs (20-25) est réalisé sous forme de trou traversant.

2. Instrument dentaire de la revendication 1,
dans lequel lesdits au moins deux trous oblongs (20-25) s'étendent en direction longitudinale.

3. Instrument dentaire selon la revendication 1 ou 2,
dans lequel lesdits au moins deux trous oblongs (20-25) sont espacés l'un de l'autre en direction longitudinale (19).

4. Instrument dentaire selon l'une des revendications précédentes,
dans lequel, pour l'un au moins desdits trous oblongs (20-25), les deux extrémités (20V, 21V, 22V, 20H, 21H, 22H) du trou oblong (20-25) sont arrondies.

5. Instrument dentaire selon l'une des revendications précédentes,
dans lequel deux desdits trous oblongs (20-25) diffèrent dans leur extension en direction longitudinale (19) et/ou dans leur extension transversalement à la direction longitudinale (19).

6. Instrument dentaire selon l'une des revendications précédentes,
dans lequel au moins deux groupes d'au moins deux trous oblongs (20-25) respectifs sont formés dans la zone de poignée (6, 10),
les trous oblongs (20-25) de chaque groupe sont disposés de manière décalée l'un par rapport à l'autre en direction longitudinale (19),
les groupes sont espacés l'un de l'autre transversalement à la direction longitudinale (19), et
lesdits au moins deux trous oblongs (20-25) de chaque groupe se trouvent sur une ligne droite.

7. Instrument dentaire selon la revendication 6,
dans lequel les groupes d'au moins deux trous oblongs respectifs s'étendent parallèlement l'un à l'autre ou forment un angle l'un avec l'autre qui est dans la plage supérieure à 0° et inférieure ou égale à 20°.

8. Instrument dentaire selon l'une des revendications précédentes,
dans lequel plusieurs rainures (27) espacées les unes des autres en direction longitudinale (19) sont formées dans la zone de poignée (6, 10) et s'étendant transversalement à la direction longitudinale (19), de sorte qu'une zone surélevée (28) est présente entre deux rainures adjacentes (27).

9. Instrument dentaire selon l'une des revendications précédentes,
dans lequel la zone de poignée (6, 10) est réalisée en forme d'arc dans la zone des trous oblongs (20-25).

10. Instrument dentaire selon l'une des revendications précédentes,
dans lequel l'un au moins des trous oblongs (20-25) présente deux côtés (22L, 22R) s'étendant parallèlement l'un à l'autre.

11. Instrument dentaire selon l'une des revendications précédentes,
dans lequel il est prévu un deuxième bras (2, 3) qui s'étend en direction longitudinale (19) et qui comprend une zone de poignée (6, 10) entre une extrémité arrière (4, 8) et une extrémité de travail avant (7, 11),
les deux bras (2, 3) sont reliés l'un à l'autre à leurs extrémités arrière (4, 8) et/ou à leurs extrémités de travail avant (7, 11), et
au moins deux trous oblongs (20, 21, 22, 23, 24, 25) sont ménagés dans la zone de poignée (10) du deuxième bras (2, 3), qui s'étendent en direction longitudinale (19) et qui sont disposés de manière décalée l'un par rapport à l'autre en direction longitudinale (19).
